# EUROPEAN PATENT SPECIFICATION

(11) **EP 1 473 019 B1**
(45) Date of publication and mention of the grant of the patent: **26.04.2006**
(21) Application number: 03425258.5
(22) Date of filing: 28.04.2003
(51) Int. Cl.: A61K 8/92, A61K 8/97, A61Q 7/00

(54) **Hair growth composition containing cork coal**
Haarwuchsmittel Korkkohle enthaltend
Composition pour la pousse des cheveux comprenant du charbon de liège

(43) Date of publication of application: 03.11.2004
(73) Proprietor: 15 & 15 Investimenti S.r.l., 20122 Milano (IT); Lopez Armada, Francisco Javier, Palma Mallorca, Baleares (ES)
(72) Inventor: Lopez Armada, Francisco Javier, Palma Mallorca, Baleares (ES)
(74) Representative: Faggioni, Marco

(56) References cited:
- US-A- 5 080 900
- US-B1- 6 210 694
- ESPEJEL RODRIGUES, M. M. ET AL.: "El uso de los encinos en la region de La Malinche, Estado de Tlaxala, Mexico" BOLETIN DE LA SOCIEDAD BOTANIC DE MEXICO, no. 64, 1999, pages 35-39, XP008022174 ISSN 0366-2128 & DATABASE BIOSIS [Online] BIOSCIENCES INFORMATION SERVICE, PHILADELPHIA, PA, US; AN: PREV199900499169, "Utilization of oaks in La Malinche region, Tlaxcala, Mexico"
- OLANSKY S: "Whole coal tar shampoo: a therapeutic hair repair system" MEDLINE, XP002086794

## Description

The present invention concerns a new type of formulation to stimulate hair regrowth in man, in particular it concerns a formulation of the above said type, which allows to achieve hair thickening and strengthening in the areas in which a hair thinning process has started.

It is a well-known fact that the problem of hair thinning and of resulting baldness affects an ever increasing number of people. Up until recently, baldness was nearly exclusively a male problem; today, however, a growing number of women too experiences premature hair loss, probably due to changed environmental and living conditions. Nowadays the incidence of this phenomenon is particularly high and the number of people affected by premature balding (i.e. onset between 30 and 60 years of age) is steadily increasing.

Baldness in itself does not imply any particular problem for the affected individual's health, as the importance of the thermo-regulating function of hair declined a long time ago, especially in Western societies in which daily activities are carried out mainly in a protected environment. However, the degree of social acceptability of the bald individual or of an individual simply affected by visible hair thinning, receding hairline, or incipient baldness, is definitely low, in particular among the young. This means that even a partly bold person often suffers from some sort of discomfort in its social dealings, to varying degrees. Such discomfort can sometimes lead to real psychological suffering that may heavily disrupt such dealings.

In the last few decades, as well as an ever increasing glorification of physical appearance, both as a means of self-gratification and as a conditioning tool of others' judgement, a further deterioration of the picture described above has taken place. It is hence not surprising that over the years a whole health market industry grew out of and has dramatically developed around the problem of baldness. Its remedies range from beauty products to surgery, the latter being an industry to which a growing number of people trustfully resorts to, but which is not always supported by positive results.

The object of the present invention is then to provide a new formulation which can find its place in this crowded scene thanks to its specific character, i.e. an extremely simple formulation and resulting low manufacturing costs, the low cost of its raw materials, the use of natural ingredients which are already largely widespread among the population, so that such raw materials are known to be free from harmful and allergic effects, a high degree of effectiveness in the treatment of the initial phases of hair thinning, and a satisfactory activity in the case of established baldness.

According to the present invention, such object is achieved by means of a hair growth formulation characterised in that it comprises a dispersion of cork coal in a natural oil or fat, and possible excipients for cosmetic purposes.

Following is a detailed description of the formulation according to the present invention, with reference to the Examples of preparation and to the Examples of application further down on the page.

### Example of preparation no. 1

100 gr. of roughly chunked, previously untreated cork (bark of Quercus Suber L.) were placed in a wide-neck laboratory flask. The flask was heated on a flame burner, thus causing partial combustion of the cork and simultaneous carbonisation of the unburnt portion.

The resulting cork coal was placed in a mortar, reduced to fine dust by means of a pestle, and mixed with olive oil obtained as direct oil flow from fresh olives crushed for the first time at room temperature (in the following, simply indicated as: first cold-crushed olive oil) until a homogeneous dispersion of a creamy consistency was obtained, ready for use.

### Example of preparation no. 2

The preparation described in the Example of preparation no. 1 was repeated using sunflower oil instead of olive oil. A Homogenous dispersion of a fluid consistency was obtained, ready for use.

### Example of preparation no. 3

The preparation described in the Example of preparation no. 1 was repeated using melted pork fat instead of olive oil. A homogeneous dispersion of a creamy consistency was obtained, ready for use.

### Example of application no. 1

The creamy dispersion obtained in the Example of preparation no. 1 described above was applied to areas of the scalp in 20 healthy males, of various ages. Such scalp areas were affected by varying degrees of baldness, from hair thinning to complete baldness. The cream was applied with a light massage in the evening, every second day for 60 consecutive days; the cream residue was removed the following morning by washing.

The first signs of hair regrowth started to show after roughly 45 days, beginning in the younger individuals. Regrowth intensity and speed were inversely proportional to the degree of baldness. As a matter of fact, at the end of the first course of application the rate of individuals aged below 40 who showed signs of hair regrowth in areas previously affected by hair thinning was about 60%. For the individuals who previously showed completely bald areas the percentage dropped to 40%. Furthermore, texture and vitality of regrown hair were noticeably improved in the former group compared to the latter. In individuals aged over 50 the percentages quoted above dropped to 40% and 20% respectively.

### Example of application no. 2

After discontinuing application of the creamy dispersion, the group of individuals who showed signs of hair regrowth was followed up over time without carrying out further applications. The follow up interviews took place at 3, 6, and 12-month intervals; during the three checks, the percentage of individuals who showed a satisfactory persistence of hair regrowth was of 80%, 60%, and 50% respectively. This showed an excellent stability over time of the positive effects of the formulation according to the present invention.

### Example of application no. 3

Within the group of individuals who, at the end of the first month of treatment, did not show any sign of hair regrowth in the treated areas, the formulation application was continued up to a total of 120 days.

At the end of this time, clear signs of hair regrowth were present in about 50% of the group, without substantial differences linked to the individuals' age. In this case too, however, the highest success rate was found in the group of individuals affected by balding in its initial stages of hair thinning.

### Example of application no. 4

A second group of 10 healthy males, aged between 30 and 60, with similar characteristics to those of the group of the Example of application no. 1, was treated according to the method described in the previous Examples of application, but using the formulation obtained in the Example of preparation no. 2. On average the results of the treatment were 5% worse compared to those of the Examples of application no. 1, 2, and 3.

### Example of application no. 5

A third group of 10 healthy males, aged between 30 and 60, with characteristics similar to those of the group of the Example of application no. 1, was treated according to the method described in the Examples 1, 2, and 3, but using the formulation obtained in the Example of preparation no. 3. On average the results of the treatment were 10% worse compared to those of such Examples of application.

### Example of application no. 6

A group of 20 healthy females, of various ages, experiencing areas of hair thinning of various intensity, was treated with the invention formulation in a similar way to that employed in the Example of application no. 1.

The first signs of hair regrowth started to show after about 35 days, beginning in the younger individuals. At the end of the first 60-day course of application, the percentage of individuals aged below 40 who showed signs of hair regrowth in the treated areas was of about 70%. In the individuals aged over 50 such rate dropped to 50%.

From the examples quoted above it is clear that, when applied to scalp areas in which a hair thinning process occurred but which did not lead to total hair disappearance, the formulation according to the present invention is effective in an extremely high percentage of cases: about 80% of cases for individuals aged below 40, and about 60% of cases for individuals aged over 50 respectively. Furthermore, the formulation effects have a sufficiently prolonged persistence to make it possible to achieve satisfyingly stable results of hair regrowth through an initial treatment of 60-120 days, followed by follow-up treatments at fairly wide-distanced time intervals, for example between 6 months and 1 year.

Also, the Examples themselves show that the formulation according to the present invention is effective with different natural oils and fats, even though the best results have so far been achieved with first cold-crushed olive oil.

Even though the Examples of preparation and of application described above refer solely to the formulation containing the active components only, it is self-evident that in the preparation of commercial formulations, additives or excipients may be added that are well-known to industry technicians. These may be added in order to give the formulation special features, without departing from the scope of the invention by doing so. In particular, those additives may include preserving agents, pH-modifying and stabilising agents, stabilisers, agents that improve skin penetration, colouring and perfuming agents, and other similar agents.

It must be also clearly understood that the present formulation may be effectively integrated in other cosmetic products to be used on the scalp or on other hair-bearing areas in which hairs should be strengthened and thickened (for example eyelashes). Hence the formulation may be integrated, to give just a few examples, in shampoos, conditioners, lotions, dyes, mascara, and other similar products. Use in such products of the formulations according to the present invention are intended to be within the scope of the invention, as defined in the appended claims.

## Claims

1. Formulation for hair growth or growth of hairs in other hair-bearing areas **characterised in that** it comprises a dispersion of cork coal in a natural oil or fat, with possible addition of excipients for cosmetic use.

2. Formulation as in 1), in which said natural oil or fat is a vegetable oil.

3. Formulation as in 2), in which said vegetable oil is olive oil.

4. Formulation as in 3), in which said olive oil is a first cold-crushed olive oil.

5. Formulation as in 1) , in which said natural oil or fat is an animal fat.

6. Formulation as in any of the previous claims, in which said formulation contains also additives suitable for giving said dispersion special properties or features.

7. Formulation as in 6), in which said additives are chosen among preserving agents, pH-modifying and stabilising agents, stabilisers, agents that improve skin penetration, colouring and perfuming agents, and other similar agents.

8. Use of a formulation as in anyone of the previous claims in a cosmetic product intended to contact hair or other hair-bearing areas.

9. Use as in 8), in which said cosmetic product is a shampoo, a conditioner, a lotion, a dye, mascara, or other similar products.

## Patentansprüche

1. Formulierung für Haarwuchs oder Wachstum von Haaren in anderen haartragenden Bereichen, **dadurch gekennzeichnet, daß** sie eine Korkkohle-Dispersion in einem natürlichen Öl oder Fett, mit möglichem Zusatz von Trägermitteln für kosmetische Verwendung, umfaßt.

2. Formulierung nach Anspruch 1, **dadurch gekennzeichnet, daß** besagtes natürliche Öl oder Fett ein Pflanzenöl ist.

3. Formulierung nach Anspruch 2, **dadurch gekennzeichnet, daß** besagtes Pflanzenöl Olivenöl ist.

4. Formulierung nach Anspruch 3; **dadurch gekennzeichnet, daß** besagtes Olivenöl ein Olivenöl aus erster Kaltpressung ist.

5. Formulierung nach Anspruch 1, **dadurch gekennzeichnet, daß** besagtes natürliche Öl oder Fett ein tierisches Fett ist.

6. Formulierung nach einem der vorangehenden Ansprüche, **dadurch gekennzeichnet, daß** besagte Formulierung auch Zusatzstoffe enthält, die geeignet sind, besagter Dispersion spezielle Eigenschaften oder Merkmale zu verteilen.

7. Formulierung nach Anspruch 6, **dadurch gekennzeichnet, daß** besagte Zusatzstoffe ausgewählt sind unter Konservierungsstoffen, pH-modifizierenden und -stabilisierenden Mitteln, Stabilisatoren, Mitteln, die Hautpenetration verbessern, Färbe- und Duftstoffen und anderen ähnlichen Mitteln.

8. Verwendung einer Formulierung nach einem der vorangehenden Ansprüche in einem kosmetischen Produkt, das dazu gedacht ist, mit Haar oder anderen haartragenden Bereichen in Kontakt zu kommen.

9. Verwendung nach Anspruch 8, **dadurch gekennzeichnet, daß** besagtes kosmetische Produkt ein Shampoo, ein Conditioner, eine Lotion, ein Färbemittel, Mascara oder andere ähnliche Produkte ist.

## Revendications

1. Formulation pour la repousse des cheveux ou la repousse des poils dans d'autres zones capillaires, **caractérisée en ce qu'**elle comprend une dispersion de charbon de liège dans une huile ou graisse naturelle, avec l'addition éventuelle d'excipients à usage cosmétique.

2. Formulation suivant la revendication 1, dans laquelle ladite huile ou graisse naturelle est une huile végétale.

3. Formulation suivant la revendication 2, dans laquelle ladite huile végétale est l'huile d'olive.

4. Formulation suivant la revendication 3, dans laquelle ladite huile d'olive est une huile d'olive de première pression à froid.

5. Formulation suivant la revendication 1, dans laquelle ladite huile ou graisse naturelle est une graisse animale.

6. Formulation suivant l'une quelconque des revendications précédentes, ladite formulation contenant également des additifs aptes à conférer à ladite dispersion des propriétés ou caractéristiques particulières.

7. Formulation suivant la revendication 6, dans laquelle lesdits additifs sont choisis parmi des conservateurs, des modificateurs de pH et des stabilisants de pH, des stabilisants, des agents qui améliorent la pénétration cutanée, des colorants et des parfums et d'autres agents similaires.

8. Utilisation d'une formulation suivant l'une quelconque des revendications précédentes dans un produit cosmétique destiné à entrer en contact avec les cheveux ou d'autres zones capillaires.

9. Utilisation suivant la revendication 8, dans laquelle ledit produit cosmétique est un shampoing, un agent conditionnant, une lotion, un colorant, un mascara ou un autre produit similaire.
